(19) **Europäisches Patentamt / European Patent Office / Office européen des brevets**

(11) **EP 1 180 549 B1**

(12) **EUROPEAN PATENT SPECIFICATION**

(45) Date of publication and mention of the grant of the patent:
**21.02.2007 Bulletin 2007/08**

(51) Int Cl.:
*C12Q 1/68* (2006.01)     *G01N 33/53* (2006.01)
*G01N 33/566* (2006.01)

(21) Application number: **01915852.6**

(22) Date of filing: **27.03.2001**

(86) International application number:
**PCT/JP2001/002495**

(87) International publication number:
**WO 2001/073120 (04.10.2001 Gazette 2001/40)**

(54) **METHOD OF MEASURING POLYMORPHISM**

VERFAHREN ZUM MESSEN VON POLYMORPHISMEN

PROCEDE DE MESURE DE POLYMORPHISME

(84) Designated Contracting States:
**DE FR GB**

(30) Priority: **27.03.2000   JP 2000087500**
**27.03.2000   JP 2000087501**
**27.03.2000   JP 2000087504**

(43) Date of publication of application:
**20.02.2002   Bulletin 2002/08**

(73) Proprietor: **Olympus Corporation**
**Tokyo 151-0072 (JP)**

(72) Inventors:
- **HORI, Kunio**
  **Chofu-shi,**
  **Tokyo 182-0023 (JP)**
- **KARAKI, Sachiko**
  **Hino-shi,**
  **Tokyo 191-0012 (JP)**
- **KANO, Tokio**
  **Kunitachi-shi, Tokyo 186-0003 (JP)**
- **TAKAMIYA, Yuji**
  **Sagamihara-shi,**
  **Kanagawa 229-0039 (JP)**

(74) Representative: **Ritthaler, Wolfgang**
**Winter, Brandl, Fürniss, Hübner,**
**Röss, Kaiser, Polte**
**Partnerschaft**
**Patent- und Rechtsanwaltskanzlei**
**Alois-Steinecker-Strasse 22**
**85354 Freising (DE)**

(56) References cited:
| | |
|---|---|
| **EP-A- 0 731 173** | **WO-A-00/49180** |
| **WO-A-94/16313** | **WO-A-97/21832** |
| **WO-A-99/09393** | **WO-A1-90/12115** |
| **WO-A2-94/16313** | **JP-A- 2000 125 900** |

- **RIGLER R: "Fluorescence correlations, single molecule detection and large number screening - Applications in biotechnology" JOURNAL OF BIOTECHNOLOGY, ELSEVIER SCIENCE PUBLISHERS, AMSTERDAM, NL, vol. 41, no. 2, 31 July 1995 (1995-07-31), pages 177-186, XP004036934 ISSN: 0168-1656**
- **KINJO M ET AL: "Ultrasensitive hybridization analysis using fluorescence correlation spectroscopy" NUCLEIC ACIDS RESEARCH, OXFORD UNIVERSITY PRESS, SURREY, GB, vol. 23, no. 10, 25 May 1995 (1995-05-25), pages 1795-1799, XP002029411 ISSN: 0305-1048**
- **KINJO M.: 'Detection of asymmetric PCR products in homogeneous solution by fluorescence correlation spectroscopy' BIOTECHNIQUES vol. 25, no. 4, 1998, pages 706 - 715, XP002942023**

**Description**

Technical Field

The present invention relates to a method for determining polymorphism, and more particularly, to a method of accurately and quickly detecting a single-nucleotide replaced site in a nucleotide sequence.

Background Art

In recent years, the link between specific disorders and gene polymorphism has been rapidly elucidated. It is expected that the analysis of gene polymorphism will be extremely useful in the screening of disease-related genes, gene diagnosis of diseases, and medical care regarding blood transfusion and transplantation.

As a method for analyzing polymorphism of a gene, a serological method and a method using DNA are primarily known. The latter method is superior to the former method in that a well-trained technician is not required for determination, and in that the examination step may be automatically performed.

However, the latter method has problems: (1) the operation is complicated and a long time is required for the analysis; (2) when a polymorphism analysis is performed by using a probe fixed onto a plate such as a micro-titer plate, items simultaneously determined are limited in number; (3) the detection cannot be performed with a sufficient accuracy since a marker substance remains and a non-specific reaction takes place; and (4) examination cost is high.

Up to the present, many types of polymorphisms have been known. Of them, analysis of single-nucleotide polymorphism (hereinafter, referred to as "SNP") has drawn attention. The SNP is defined as a polymorphism in which a single nucleotide of the nucleotide sequence is replaced. In the case of humans, the homology of genome DNAs between individuals is 99% or less. The difference is as low as about 1%. For this reason, concern for the SNP has been increased.

As a method for detecting SNP, a wide variety of methods are known including PCR-RFLP, PCR-SSP and PCR-SSO. PCR is employed in all of these methods. In these methods, a PCR product is electrophoretically analyzed and hybridized with a probe sequence.

For example, in the PCR-SSP method, a sequence-specific primer reagent is used for specifically amplifying a polymorphism site. The method is frequently used for SNP determination. However, in this method, after the amplification, electrophoresis must be performed in order to confirm the presence of side product(s).

Furthermore, another method is known for detecting SNP. In this method, SNP is detected by a VDA technique (high-density variant detection array) using a device such as a DNA chip or a DNA microarray (Science Vol. 280, May 15, 1998). In the VDA technique, a plurality of probe DNAs are densely arranged on the surface of a solid-phase substrate and sample DNA is allowed to hybridize with the probe DNA(s) on the solid-phase surface. However, the efficiency of this method is considered low.

In addition, since Tm values of polymorphism sites are almost the same in the case of SNP, it is difficult to accurately detect a mismatch by conventional methods. For this reason, detection cannot be performed with an accuracy sufficient for clinical trials in conventional methods.

WO 94/16313 discloses the use of fluorescence correlation spectroscopy (FCS) in a method of detecting a polymorphism site, wherein a test sample containing a polymorphism site is reacted with a plurality of probes specific for various polymorphism sites and labelled with the same fluorescent marker. A further probe which is labelled with a second fluorescent marker hybridizes to the target but not to a polymorphism site. Detection of cross-correlation signals between the differently labelled probes indicates the presence of at least one of the polymorphisms tested.

R. Rigler, Journal of Biotechnology 41:177-186, 1995, discloses FCS and its use for detection of antigen-antibody interactions.

Disclosure of Invention

An object of the present invention is to provide a method for analyzing polymorphism quickly, easily, and accurately. In particular, an object of the present invention is to provide a method of sensitively and accurately analyzing polymorphism even if polymorphism occurs at a site of a single-nucleotide.

The object is directed to a method of detecting a polymorphism site, comprising:

(1) reacting, in a same vessel, a test sample containing a polymorphism site with a plurality of types of probes corresponding to a plurality of types of the polymorphism site to be identified of said test sample, said probes binding to said plurality of types of the polymorphism site with a high affinity and being labelled with marker substances so as to optically distinguish from each other; and
(2) optically measuring and analyzing a positional change of the marker substances by micro-movement at a plurality of time points in the course of the reaction, thereby detecting the types of polymorphism sites of said test sample.

According to an aspect of the present invention, there is provided a polymorphism analysis method which uses smaller amounts of reagent and test sample than a conventional method.

According to another aspect of the present invention, there is provided a method in which a plurality of polymorphism sites can be simultaneously detected by using small amounts of test sample and reagent. According to still another aspect of the present invention, there is provided a method for quickly and easily determining an extremely large number of polymorphism sequences. Since B/F isolation, PCR, electrophoresis, and the like are not required by these methods, various types of polymorphisms can be easily analyzed.

From the idea of the present invention as mentioned above, the following effects can also be obtained. To be more specific, the present invention enables determination not only of a single-nucleotide replaced site but a nucleotide sequence of a polymorphism site. Furthermore, it is possible to easily detect blood-cell polymorphism such as a blood type.

Brief Description of Drawings

FIG. 1 is a schematic illustration of test sample DNA detectable in a detection method according to Example 1 of the present invention;

FIG. 2 is a schematic illustration of a probe which can be used for detecting the test sample DNA of FIG. 1;

FIG. 3 is a scheme showing a general outline of a reaction of a probe I in a detection method according to Example 1 of the present invention;

FIG. 4 is a scheme showing a general outline of a reaction of probe II in a detection method according to Example 1 of the present invention;

FIG. 5 is a scheme showing an example of a detection system which can be used in a detection method according to an embodiment of the present invention;

FIGS. 6A and 6B show an example of a measurement portion of a fluorescent microscope to be used in an embodiment of the present invention;

FIG. 7 is a graph showing a time-dependent change of fluorescence intensity to be obtained by the detection system shown in FIG. 5;

FIG. 8 is a graph showing statistical data obtained by converting the data shown in FIG. 7 by an autocorrelation function;

FIGS. 9A-9D are view showing the outline of a method for determining a polymorphism sequence by a detection method according to an embodiment of the present invention; FIG. 9A shows the movement of relatively small molecules in a micro-space, FIG. 9B is a graph showing fluctuation with time in the case of FIG. 9A, FIG. 9C shows the movement of relatively large molecules in a micro-space, and FIG. 9D is a graph showing fluctuation with time in the case of FIG. 9C;

FIG. 10 shows a probe to be used in Example 3 of the present invention;

FIG. 11 shows an application example of a method of the present invention in clinical medicine;

FIG. 12A is a schematic view of a sample dotted on a slide glass, and FIG. 12B is a cross-sectional view taken long the line XIIB-B of FIG. 12A; and

FIGS. 13A to 13C are examples of microplates to be used in Example 4 of the present invention, and FIG. 13D is a cross-sectional view taking along the line XIIIC-C of the microplate shown in FIG. 13C.

Best Mode for Carrying Out of the Invention

In generally, the method for determining polymorphism according to the present invention roughly comprises two steps, namely, a reaction step and a detection step. The reaction step is one of reacting a probe substance capable of specifically binding to a desired polymorphism site with the polymorphism site to bind them. The detection step is a process for determining the movement of molecules during or after the reaction step.

Terminology

In this text, the term "polymorphism" refers either to a allele group containing a plurality of types of alleles occupying a single genetic locus or to individual alleles belonging to the allele group.

The term "polymorphism site" used herein is a site whose nucleotide sequence differs between polymorphism genes. For example, if the nucleotide sequence of a polymorphism gene A1 is AAA TTT (CCC) GGG and the nucleotide sequence of a polymorphism gene A2 is AAA TTT (AGT), GGG, the site bracketed by parentheses corresponds to the polymorphism site. If a single nucleotide differs in the polymorphism site, such a polymorphism is particularly designated as a "single nucleotide polymorphism".

According to an embodiment of the present invention, the polymorphism to be detected is not limited to the polymorphism of a gene level. The polymorphism of a protein level is also included. The "polymorphism site" of this case indicates a protein expressed.

The term "single-nucleotide replaced site" used herein refers to a polymorphism site consisting of a single nucleotide, from which the single-nucleotide polymorphism is derived.

According to an embodiment of the present invention, if the single-nucleotide replaced site is present either singly or in a plurality of numbers in the sequence of a single test sample DNA, it (or they) can be detected. When a plurality of single-nucleotide replaced sites are detected, a plurality of types of marker substances may be used.

The term "polymorphism sequence" used herein refers to a nucleotide sequence contained in a polymorphism site. In the example shown above, the "polymorphism sequence" is the nucleotide sequence bracketed by parentheses.

The term "a nucleic acid synthetase having a repair function" used herein refers to an enzyme which recognizes a mismatch at the 3' end of the nucleotide sequence partially double stranded, and deletes the mismatched nucleotide, and then, synthesizes a nucleic acid under appropriate conditions to complete the double strand. According to an embodiment of the present invention, examples of usable enzymes include nucleic acid synthetase. Preferably, Ex/Taq or La/Taq is used. These enzymes can be available from, but not limited to Takara Shuzo Co., Ltd.

The term "marker substance" used herein refers to a marker emitting a signal with much the same intensity at any measuring time-points. For example, luminescent substances, fluorescent substances, magnetic substances, and radioactive materials are included. Note that a substance suitable for a means to be used in the detection step (described later) should be selected as the marker substance.

The term "free micro-movement" or "micro-movement" used herein primarily refers to the Brownian movement.

The term "micro-space" used herein refers to a small space in which the free micro-movement of molecules can be satisfactorily detected. The micro-space to be used in accordance with an embodiment of the present invention may have a volume of $10^{-21}$L (corresponding to the volume of a cube of 100 nm each side) to $10^{-3}$L. The micro-space may typically have a volume of $10^{-18}$L to $10^{-9}$L, and most typically, $10^{-15}$L to $10^{-12}$L. The micro-space may take any shape including a sphere, cylinder, cone, cube, or rectangular parallelepiped.

According to an aspect of the present invention, there is provided a method for determining a nucleotide sequence contained in a polymorphism site of an arbitrarily chosen polymorphism gene. In other words, there is provided a method of determining the genotype of a polymorphism gene.

Hereinafter, polymorphism sequences known to be contained in a polymorphism site of a polymorphism gene to be detected (hereinafter, referred to as a "target polymorphism gene") are represented by $PS_1$ to $PS_n$ (n is an integer of 2 or more). In the aforementioned example, $PS_1$ is, for instance, CCC and $PS_2$ is AGT.

If the method according to an embodiment of the present invention is used, the type of a polymorphism site can be quite quickly determined. Furthermore, according to an embodiment of the present invention, it is possible to detect a polymorphism gene containing a plurality of polymorphism sites and a polymorphism gene having a plurality of types of polymorphism sites. Examples of polymorphism gene include, but not limited to various SNPs, major histocompatible antigens containing a human leukocyte antigen (hereinafter referred to as "HLA"), and various types of disease-associated genes.

According to an embodiment of the present invention, when a polymorphism site is detected or the type of a polymorphism site is detected, a test sample containing either a target polymorphism gene or a protein is first prepared. According to an embodiment of the present invention, when such a method is applied to humans, the test sample may be, but not limited to a body fluid including blood, spinal fluid, and cerebrospinal fluid.

According to an embodiment of the present invention, it is possible to detect polymorphism with a high sensitivity. Therefore, when a gene is used, a conventional PCR amplification operation may be omitted. However, if a target polymorphism gene is present in a small amount, PCR amplification may be performed.

Example 1

According to an aspect of the present invention, there is provided a method of detecting a single nucleotide polymorphism site in a nucleotide sequence.

The reaction step previously mentioned generally comprises hybridizing test sample DNA with a labeled DNA probe and treating the resultant double strand with an enzyme having a repair function.

In the detection step, generally, a free micro-movement of molecules taking place in a reaction system of the reaction step is measured and evaluated.

1. Reaction step

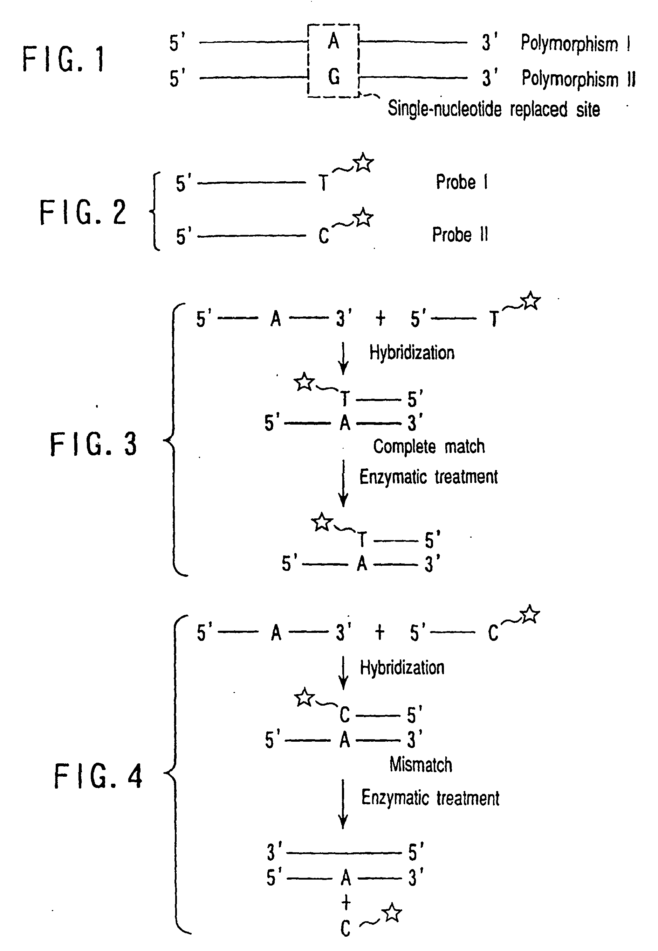

Now, a reaction step of this example will be explained with reference to FIGS. 1 to 3. However, it is an example, so that it will not limit the present invention.

(1) Test sample DNA

FIG. 1 is a schematic illustration of test sample DNA of this example. In this figure, a polymorphism gene of the test sample DNA has two polymorphism sequences, namely, the polymorphism is present as polymorphism I or polymorphism II. Polymorphism I and polymorphism II are homologous except a single-nucleotide replaced site. Furthermore, the single-nucleotide replaced site of polymorphism I is adenine (hereinafter, referred to as "nucleotide (A)" or "A" as shown in the figure). The single-nucleotide replaced site of polymorphism II is guanine (hereinafter, referred to as "nucleotide (G)" or "G" as shown in the figure).

(2) Labeled DNA probe

Now, a probe for detecting the single-nucleotide replaced site (shown in FIG. 1) is shown in FIG. 2. Probe I is composed of a sequence complementary to the sequence from the 3' end to the single-nucleotide replaced site of polymorphism I. In addition, a marker substance is attached to the nucleotide of the 3' end of probe I, (that is, the nucleotide paired with the nucleotide of the single-nucleotide replaced site). In this example, the 3' end of probe I is a labeled thymine (hereinafter, referred to as "(T)" or "T" as shown in the figure).

Similarly, probe II is composed of a complementary sequence to the sequence from the 3' end to the single-nucleotide replaced site of polymorphism II. In addition, a marker substance is attached to the nucleotide of the 3' end of probe II. In this example, the 3' end of probe II is a labeled cytosine (hereinafter referred to as a "nucleotide (C)" or "C" as shown in the figure).

(3) Reaction step

As the simplest example of the present invention, a reaction in a reaction system of the method according to Example 1 is illustrated in FIGS. 3 and 4. Now the reaction will be simulated. In this example, it is determined that test sample DNA contained in a sample is either polymorphism I or II. In this case, on the assumption that the sample contains polymorphism I, the sample is first placed in two vessels, namely, vessel I and vessel II.

Probes shown in FIG. 2 are placed separately in vessels varied depending upon types, and subjected to reactions with the sample. FIG. 3 shows a reaction performed in vessel I. To explain more specifically, in vessel I of FIG. 3, hybridization of polymorphism I with probe I proceeds under optimum conditions. As is apparent from FIG. 3, polymorphism I is completely matched with probe I (FIG. 3). On the other hand, a reaction performed in vessel II is shown in FIG. 4. More specifically, in vessel II of FIG. 4, polymorphism I is hybridized with probe II under optimum conditions. As is apparent from FIG. 4, a mismatch is observed at the single-nucleotide replaced site.

Subsequently, DNA polymerase having a repair function is added to each of the completely matched double strand and the mismatched double strand (FIGS. 3 and 4). As a result, the polymerase does not exhibit an activity (namely, 3' → 5' exonuclease activity) to the completely matched double strand of vessel I (FIG. 3).

In contrast, the polymerase of vessel II recognizes a mismatch of the 3' end of the double strand formed of polymorphism I and probe II, and delete the mismatched nucleotide, and further extends the DNA strand (FIG. 4). As a result, the labeled nucleotide (C) is liberated in vessel II (FIG. 4).

More specifically, when DNA polymerase serving as a nucleic acid synthesizing reagent is reacted to a hybridization reaction product which possibly have a local mismatch, the following two reactions may occur.

In the case where the hybridization reaction product has a mismatch, the DNA polymerase recognizes the mismatch. As a result, the extension reaction of the DNA strand is advanced by the DNA polymerase. At the same time, the marker molecule including a mismatched nucleotide is dissociated from the target DNA in the course of the extension reaction.

On the other hand, in another case where the reaction product is a completely matched double strand without a mismatch, in other words, the case where a target DNA is completely hybridized and matched with a nucleotide having a marker molecule, the DNA polymerase recognizes that the double strand is completely matched and bonded. As a result, the DNA polymerase does not cause an extension reaction. Even if the extension reaction takes place, the extension is terminated in the middle. The extension reaction will not proceed any more. Since a mismatch is not present, the exonuclease activity of the DNA polymerase, which is related to a repair function, will not be exhibited. Such a unique idea of the present inventors, that is, that idea that the repair function of the DNA polymerase is used for removing a marker molecule containing a mismatch nucleotide, greatly contributes to providing a quite novel and extremely effective method for detecting polymorphism.

In the case where the detection is performed by use of the repair function of DNA polymerase, detection is performed in two stages: detecting the presence or absence of hybridization and detecting dissociation or nondissociation of a marker molecule by the repair function. Therefore, it is possible to provide a more accurate detection method.

In other words, the two-stage detection comprises the following two steps. In a first detection step, a sample and a marker molecule are mixed and reacted under the conditions causing hybridization. The presence or absence of hybridization during the reaction is detected. In this manner, the presence or absence of the target DNA and/or a reaction amount of hybridization can be determined. Subsequently, in the second detection step, the nucleic acid synthetase is added and reacted in the conditions under which a synthetic reaction may take place. The dissociation molecule generated in this step is detected. In the manner mentioned above, the presence or absence of a genetic mutation is detected and/or a mutated sequence can be determined.

The two-stage detection step consisting of the first and second detection steps may be performed by FCS either continuously or separately. In the case where the two-stage detection is performed by FCS, the following results can be obtained. When the hybridization occurs in the first detection step, the size of a marker molecule increases, varying a FCS reaction curve. Subsequently, in the second detection step, when the dissociation occurs, the size of the marker molecule decreases. As a result, the reaction curve which has been changed in the previous step further varies. As described, when the repair function of the DNA polymerase is used, the presence or absence of hybridization is first detected. Second, whether or not the marker molecule is dissociated by the repair function, is detected. Therefore, it is possible to provide a significantly accurate detection method having a more excellent reliability than a conventional method.

Note that according to an embodiment of the present invention, the mismatch detection, which is performed by a reagent such as DNA polymerase having a repair function, is not always performed by a method employing FCS as a detection means. As the detection means, use may be made of various B(bound)/F(free) separation techniques in which separation is performed based on binding or nonbonding. In this case, detection can be performed by comparing the amounts ($B_1$) and ($B_2$) of marker molecules bound to the target DNA, where the amount $B_1$ and B2 are obtained in the respective stages of the two-stage detection method. Alternatively, detection can be made by comparing the amount ($B_1$) with the amount ($F_2$), which is the amount of the marker molecule dissociated. Herein, ($B_1$) is the amount of the marker molecules contained in the probe hybridized with the target DNA obtained after the first detection step. ($B_2$) is the amount of the marker molecules contained in the probe hybridized with the target DNA obtained after the second detection step. ($F_2$) is the amount of the marker molecules dissociated after the second detection step.

The free micro-movement of marker molecules obtained by the aforementioned reaction(s) is determined and evaluated by the detection means described later. The free micro-movement of the target molecule can be varied depending upon the size of the marker molecule. Therefore, it is possible to obtain information on the nucleotide sequence by measuring the micro-movement of the marker molecules by using as the marker substance as an index.

(4) Another embodiment of the reaction step

The simplest example of the reaction step included in the detection method of the present invention is described above. However, various changes and modifications can be made. For instance, a plurality of marker substances may be used. A marker substance may be attached to a probe at not only the 3' end but also the 5' end. A plurality of probes may be reacted with a sample in a single vessel.

Furthermore, after a mismatch is recognized or dissociated by a nucleic acid synthetase having a repair function, the nucleotide sequence can be extended from a cleaved site toward the 3' end. In this case, the extension may be performed by supplying a requisite substrate and a reagent to be further required, under optimum conditions. Alternatively, the extension may be performed by a polymerase chain reaction (hereinafter referred to as PCR). However, the extension may not be always performed.

Moreover, a polymorphism site contained in a sample may be amplified by, for example, a PCR technique before the aforementioned reaction(s) are performed.

2. Detection step

(1) Detection principle

In the detection step of the present invention, the output signal intensity of marker molecules (one or more molecule) is measured in a predetermined space. Furthermore, the increase or decrease of the output signal intensity thus obtained is employed as an index. Based on variation of the output signal intensity, it is possible to obtain the moving speed of the marker molecules moving in or out of a micro measurement field of view. Therefore, the marker molecule to be labeled to a probe is preferably a marker material which can output a signal with a constant intensity at any time points. Examples of such a marker molecule include luminescent substances, fluorescent substances, magnetic substances and radioactive materials. As the luminescent substances and fluorescent substances, a dye capable of emitting luminescence or fluorescence for a long time is preferably selected.

Examples of fluorescent dyes to be used as a marker include various known materials such as DAPI, FITC, rhodamine, Cy3, CY3.5, Cy5, Cy5.5, and Cy7.

For example, to detect a plurality of items at the same time, a plurality of fluorescent substances may be simultaneously used.

If a material containing a luminescent dye or fluorescent dye is used as the marker molecule, an optical determination of a molecular condition can be made at a molecular level by a device of a simple structure. Furthermore, when nucleotide molecules are complementarily bound to each other in the hybridization performed in Example 3 described later, use may be made of a substance capable of being intercalated in the complementary binding portion, thereby varying its fluorescent characteristics from those of a free state. Examples of such a fluorescent dye include acridine orange, thiazole orange, oxazole yellow, and rhodamine.

When the positional change of a fluorescent molecule is measured, fluorescence may be detected in the form of data by using a fluorescent measurement means such as a photomultiplier or a photodiode serving as a detection means. Furthermore, the fluorescent measurement means may have a measurement mode capable of detecting a single photon in order to measure individual fluorescent molecules.

According to an embodiment of the present invention, the detection means to be used in the detection step comprises a measuring means capable of measuring a signal emitted from a marker molecule depending upon the material of the marker molecule, a memory means for storing a plurality of measurement data items obtained by the measuring means in a predetermined time, and an operation means for operating the stored measurement data items by an autocorrelation function.

The detection means may further comprise the followings: a memory means for storing measurement data items regarding a plurality of marker molecules obtained in a predetermined measuring area and a calculation means for calculating the stored measurement data items separately per marker molecule by an autocorrelation function. Furthermore, an output means may be provided for outputting the obtained data or analytic results. The data output means may include a conversion means for converting the results obtained by the autocorrelation function into statistical data, which express a positional change with time with respect to a plurality of data items monitored.

In the detection step of this example, the micro-movement of the target molecules may be accurately measured and evaluated by measuring the fluctuation movement of the marker molecules in a liquid. The evaluation of the fluctuation movement may be performed through the operation using the autocorrelation function. When a fluorescent substance is used as the marker molecule, fluorescence correlation spectroscopy (hereinafter simply referred to as "FCS") may be employed.

As to the operation method for analyzing data obtained by measuring characteristics of a biological material by FCS, the report of Kinjo et al. can be referred to (Kinjo, M., Rigler, R., Nucleic Acids Research 23, 1795-1799, 1995). The document reports the case where the hybridization reaction between a labeled nucleic acid probe and a target nucleic acid molecule is observed by FCS. FCS and operation of measurement data will be more specifically described later.

If FCS is used, it is possible to obtain the number of fluorescent particles contained in an extremely small volume of a sample and a diffusion constant almost in real time without a separation step. If the FCS is used, B/F separation is not required, so that the measurement time can be reduced. Since a solution system can be subjected as it is to the measurement, the measurement time can be further reduced. In addition, a biological molecule can be measured under natural conditions.

The detection of the marker molecules obtained in the reaction step mentioned above may be carried out by the following step. The detection step according to Example 1 of the present invention comprises microscopically observing the movement of the marker molecules in a micro field of view, converting a plurality of measurement data items into time-dependent statistical data, and obtaining a reaction curve of a hybridization reaction based on the statistical data. In the reaction curve, the initial height of the curve represents the number of nucleotide molecules.

The detection step of the present invention is performed in a three-dimensional micro field of view. By virtue of this, a free micro movement of the target molecules in a sample-containing solution can be detected and evaluated with a high accuracy. Assuming that the detection step is performed by carrying out the measurement in a two-dimensional field of view, it is impossible to capture a three-dimensional free movement, such as the Brownian movement, of the marker molecules. As a result, the measurement accuracy becomes low. Therefore, such a detection is not preferable.

Since the micro field of view employed in the detection step is formed by a confocal optical system, measurement data having a deep depth-of-field can be obtained. By virtue of this, some of individual marker molecules always come into a focus in a field of view, so that an accurate portion and output data can be obtained.

The detection means for detecting the movement of micro molecules preferably has a means having an optical system in order to measure the movement in a micro field of view which is set in a diffraction-limited region near a focal point. Alternatively, a detection means preferably has a microscope to perform measurement in a micro field of view formed by the confocal optical system.

Since the micro field of view is a diffraction-limited region near a focal point, the individual marker molecules can be measured in a high S/N ratio.

The diffraction-limited region is formed by a pin hole of $15 \pm 5$ $\mu$m in average diameter. Therefore, the measurement data of a small number of marker molecules selected can be efficiently obtained.

A particularly preferable micro space is nearly a cylindrical region having an average diameter of $200 \pm 50$ nm and an average diameter on the optical axis of $2000 \pm 500$ nm. It is therefore possible to capture a free micro movement of the marker molecules brought into within the measurement field of view.

Furthermore, the micro field of view can be obtained by such an optical design that light beams are emitted from an aperture (pin hole, optical fiber end surface, etc.) of an extremely small average diameter). Converged light of laser beams is preferable.

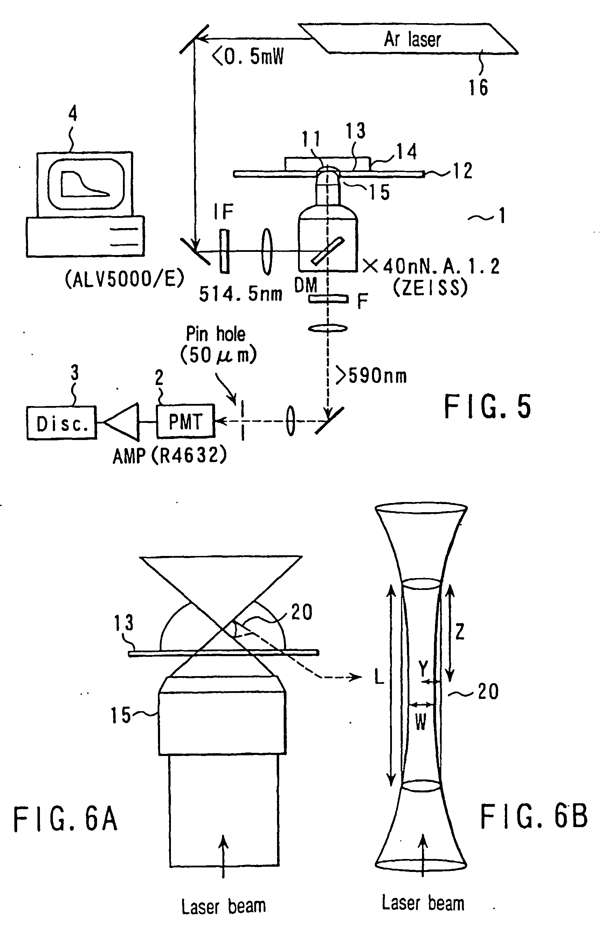

The laser light, which is excitation light, is focused on only a single point of a sample solution. The fluorescent light emitted from the point can be captured by a detection system due to the characteristics of the confocal optical system. Actually, the measurement region of a vessel is not an ideal point but a cylindrical region shown in FIG. 6B. Any size (volume) of measurement region may be used as long as it can form a micro space. For example, the size may be about 500 nm (diameter) $\times$ 2000 nm (axial length). The volume may be in the order of femto-litle.

The FCS measurement region is a solution. The fluorescent molecules present in the region move in accordance with the Brownian movement. Therefore, the number of molecules present in a predetermined measurement region is not always constant. The number of molecules fluctuates up and down from a certain value. This is called as "fluctuation in the number". Furthermore, due to the fluctuation in the number, the intensity of the measured fluorescence fluctuates. This is called "fluctuation in fluorescence intensity". By analyzing the fluctuation in fluorescence intensity, data for diffusion speed and the number of molecules can be obtained.

FCS itself is known well. The FCS is described in detail in Patent Application No. 10-301316. The FCS data analysis may be performed by an analytic software "FCS ACC ESS" attached to ConfoCor (registered trademark) manufactured by Zeiss Co., Ltd.

Example 2

An example of a measuring device to be used in the embodiment described in Example 1 will be explained with reference to FIG. 5. FIG. 5 shows an FCS device according to an embodiment of the present invention. The device comprises an inverted fluorescent microscope 1 using a confocal optical system, a photomultiplier 2 for measuring fluorescence emitted from a sample, a data processing device 3 for receiving measurement data and calculating them by an autocorrelation function to convert them into numerical values or graphic plots, and a display unit 4 for displaying the operation results on a screen.

A sample-containing solution 11 is easily set, as shown in FIG. 5, in such a manner that the solution 11 is dotted on a slide glass 13 placed on a sample base 12. Since a small amount of sample-containing solution is used in this device, a cover 14 is placed on the slide glass 13 in order to prevent a moisture content from vaporizing. A low light-transmissible material is preferably used as the cover 14. Air-tightness and light-tightness can be simultaneously ensured by the presence of the cover. It is further preferable that a material having a light reflectivity as low as possible be used as the inner surface of the cover in order to prevent excitation light from reflecting.

An objective lens 15 is arranged right under the portion of the slide glass 13 on which the sample-containing solution 11 is dotted so as to focus within the sample-containing solution 11. Note that the fluorescent microscope 1 may be a reflection type. In the reflection type, the sample-containing solution 11 may be dotted directly on the lower surface of the objective lens 15. In the example shown in FIG. 5, an argon (Ar) ion laser is used as a laser-generating device 16, which is a light source of the fluorescent microscope 1.

If necessary, various operations such as the loading/unloading of the slide glass 13 into/from the fluorescent microscope 1, dotting of a sample-containing solution onto the slide glass 13 and open/shut of the cover 14 may be performed automatically.

The aforementioned detection can be performed by a confocal microscope as shown in FIG. 5. The confocal microscope itself has been known in the art.

The detection can be performed by use of the confocal microscope, comprising

(1) applying laser light as excitation light.
(2) passing laser light through a filter (IF), converging and applying it to a single point of a sample by use of a dichroic mirror (DM);
(3) exciting a fluorescent substance in the sample by the laser light to emit fluorescence;
(4) passing the fluorescence through a pin hole to amplify fluorescence only emitted from the fluorescent substance within the focus of the sample by a multiplier phototube (PMT); and

(5) detecting the fluorescence thus amplified.

FIG. 5 shows an example of an argon ion laser. Depending upon a type of a fluorescent substance, a krypton-argon ion laser, helium-neon laser, helium-cadmium laser different in wavelength may be used. FIG. 5 is only a schematic view of a typical confocal microscope. It is needless to say that a system other than the confocal microscope shown in FIG. 5 may be used.

In the detection method, only fluorescence emitted from a single and micro point is detected. Therefore, the detection can be carried out without background and the detection sensitivity is significantly high compared to the fluorescent detection usually performed.

The detection of a target polymorphism gene is performed for a time-period generally in the order of milli-second to minute, and most generally, in the order of second.

After the detection results are obtained, the results are analyzed to determine which nucleic acid probe binds to the target polymorphism gene. If the type of nucleic acid probe bound to the target polymorphism gene is determined, the type of the target polymorphism gene can be elucidated.

FIGS. 6A and 6B are magnified views of a measurement portion of the fluorescent microscope 1 of FIG. 5. In FIG. 6A, a micro space 20 is formed which is defined by the positions of the slide glass 13 and the objective lens 15 having a predetermined aperture (FA=1.2 in the figure). As shown in FIG. 6B, the micro space 20 is actually a focal point of laser light having a volume. The shape of the micro space 20 is nearly a cylindrical shape stretched up and down from a constricted middle portion (see FIG. 6B). The field of vision 20 is restricted by the length Z on the optical axis and an average radium Y based on the reference position as a focal point. In the micro space 20, fluorescence of individual fluorescent molecules can be accurately measured. This is because the volume of the micro space 20 is reduced to the minimum sufficient to monitor the micro movement of the fluorescent molecules. With this structure, it is possible to remove the noise derived from the fluorescent molecules and present except the vicinity of the focal point of the sample-containing solution 11.

Now, an analysis using the aforementioned FCS device will be explained.

When the fluorescence intensity of fluorescent molecules moving in and out of the confocal region (as shown in FIG. 6) is captured per molecule, the fluctuation of fluorescence intensity can be detected. Furthermore, when the fluctuation of the fluorescence intensity obtained in the form of data is converted by an autocorrelation function, statistical data is given. In this way, the number and sizes of fluorescent molecules can be evaluated without isolating the molecules.

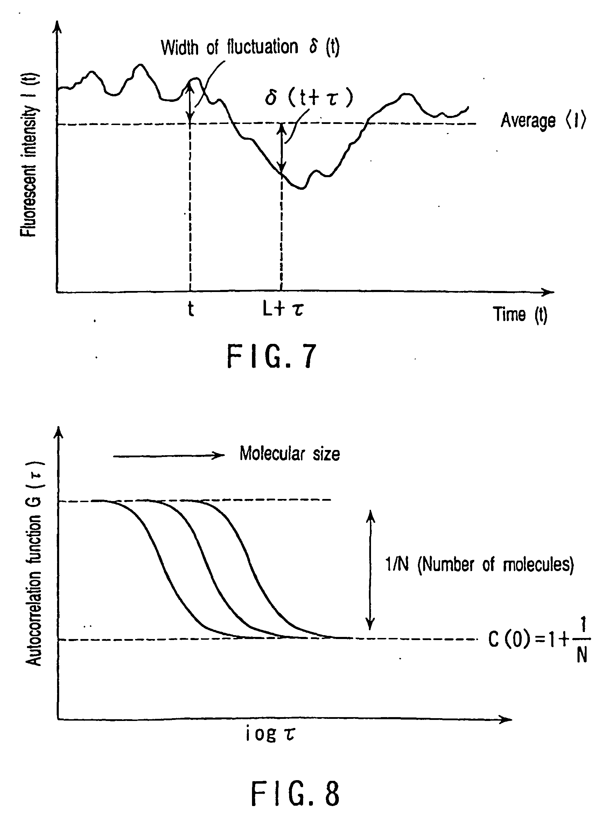

An example of data measured in accordance with the aforementioned embodiment is shown in the form of a graph of FIG. 7. The graph of FIG. 7, the fluorescence intensity I(t) is plotted on the vertical axis and time (t) is plotted on the horizontal axis. When the data is converted by an autocorrelation function shown below, the graph shown in FIG. 8 is given.

The autocorrelation function is

$$G(\tau) : G(\tau) = <I(t)I(t + \tau)>$$

When this is normalized by the square of average intensity <I> and developed, the following approximation is given:

$$G(\tau)/\langle I \rangle^2 = C(\tau)$$

$$= 1 + \frac{1}{N}\left[\frac{1}{1 + 4D\tau/w_{xy}^2}\right]\left[\frac{1}{1 + 4D\tau/w_z^2}\right]\frac{1}{2} \quad \ldots (1)$$

Where c(o)= 1+1/N in which $\tau$=0, D = diffusion constant, N = the number of molecules in a solution.

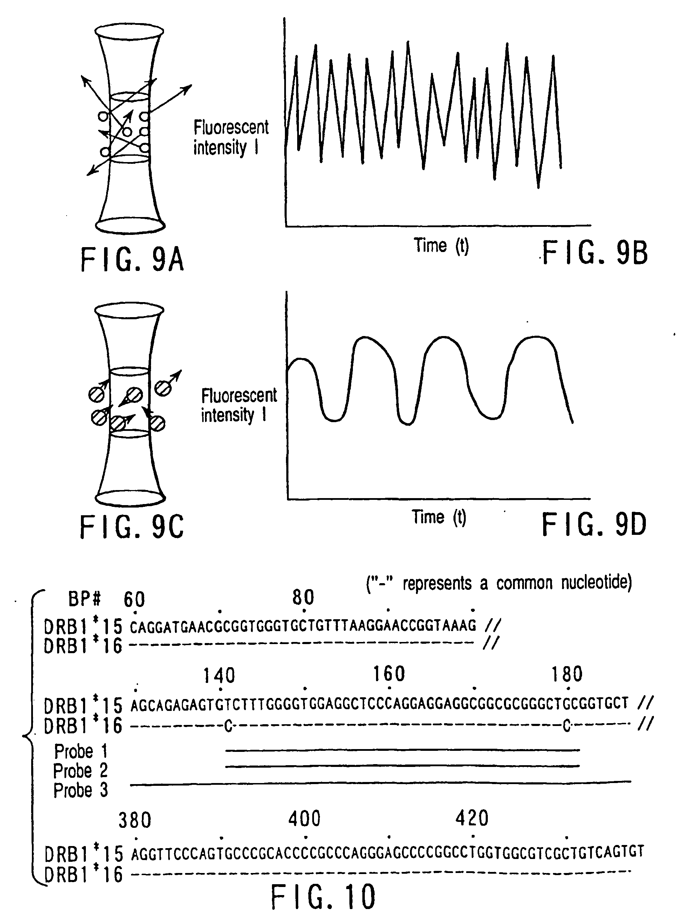

The concept of the analysis will be understood if referred to FIGS. 9A to 9D. More specifically, when the marker molecule is not bound, the size of the molecule is small. As a result, the speed of the Brownian movement is high (FIG. 9A) and the frequency is large in the function I (t) (FIG. 9B). In contrast, when the marker molecule is bound, the speed of the Brownian movement becomes low (FIG. 9C). As a result, data of a large frequency can be obtained (FIG. 9D). Hence, if analysis is made on the autocorrelation function obtained on the basis of the fluorescence intensity as mentioned above, the state of the marker molecules can be elucidated.

According to another aspect of the present invention, the embodiments mentioned above can be modified as described below.

A marker substance different in fluorescent wavelength may be attached to the nucleotide molecule of the 3' end of the probe depending upon the type of nucleotide sequence of the polymorphism site. The probe thus prepared is mixed with a test sample DNA to perform hybridization in the same manner as in Example 1. The nucleotide mismatched with the nucleotide sequence is enzymatically removed. Subsequently, detection is performed by FCS. At this time, individual marker substances are analyzed based on wavelengths different from each other. By the analysis, it becomes apparent which probe is completely matched with the nucleotide sequence. At the same time, the type of the nucleotide at the 3' terminal is identified. It is there determined which type of polymorphism is present. As a result, the single nucleotide replacement can be more reliably detected. Furthermore, when another type of fluorescence is used, the wavelength of the excitation light may be changed. Alternatively, the wavelength of the detection light may be changed by attaching a filter to the detection portion.

In the foregoing, an example of the FCS device is described. However, the present invention will not be limited to the aforementioned example. Each of the contents of the embodiments of the present invention can be modified and altered.

According to an aspect of the present invention, there is provided a single nucleotide detection method simply and quickly performed. Such an advantage is derived from the principle of the present invention originally found. More specifically, desirable detection of the present invention can be performed by labeling each of polymorphism-specific probes with a marker, reacting the probe with a test sample DNA, and detecting the marker. In this way, the reaction can be captured at a molecular level. At this time, the marker substance is measured at a plurality of time points, thereby detecting and evaluating the time-dependent positional change. Consequently, the positional change, which is varied depending upon the size of the molecule, can be quantitatively determined.

It is therefore not necessary to carry out operation steps required by conventional methods including a B/F separation step, a reaction step of a substrate with an enzyme marker reagent, an exposure step of an RI marker reagent to a radiation-sensitive film, a PCR step, and an electrophoresis step.

To explain more specific, conventional methods requires PCR and to extend the nucleotide sequence up to 200 to 300 nucleotides in order to obtain clear results from electrophoresis. However, electrophoresis and PCR are not always needed according to an embodiment of the present invention. If the PCR is performed to improve the reliability, a sufficiently reliable data can be obtained by extending the nucleotide sequence up to a length of 10 to 30 bases.

The detection method according to an embodiment of the present invention can be performed by using extremely small amounts of test sample and reagent. More specifically, it is sufficient if the test sample is contained in the order of femto liter (fL). Therefore, according to an embodiment of the present invention, it is possible to simultaneously measure a plurality of test samples by using an extremely small vessels. Furthermore, it is possible to reduce the amounts of test sample and detection reagent, such as an enzymatic reagent, to be required for detection.

Furthermore, according to an embodiment of the present invention, a plurality of reagents having specificities to different polymorphism sites can be mixed in the same vessel and therefore simultaneously applied to the test sample. As a result, not only the amount of sample DNA but also the number of reaction vessels can be reduced. In addition, a polymorphism gene test can be easily performed.

According to an embodiment of the present invention, a single nucleotide replacement can be attained with a high sensitivity. Since the method of this example mentioned above is performed in a homogeneous system little affected by the background, the detection accuracy is high. This is based upon the detection principle of the present invention.

Example 3

According to an another aspect of the present invention, there is provided a method for detecting polymorphism of a nucleotide except for SNP.

More specifically, this embodiment provides a method of detecting polymorphism, comprising the steps of preparing a test sample containing a polynucleotide;

mixing nucleic acid probes $PR_1$ to $PR_n$ labeled with a detectable marker and capable of specifically binding to polymorphism sequences $PS_1$ to $PS_n$, with the test sample; thereby binding the nucleic acid probes $PR_1$ to $PR_n$ to the polynucleotide;

detecting the nucleic acid probes $PR_1$ to $PR_n$ present in a micro space; and

analyzing the detection results to determine which one of nucleic acid probes $PR_1$ to $PR_n$ is bound to the polynucleotide, thereby determining which of the polymorphism sequences $PS_1$ to $PS_n$ corresponds to the nucleotide sequence of the polymorphism site.

According to this embodiment, it is possible to determine which of the polymorphism sequence $PS_1$ to $PS_n$ (n is an integer 2 or more) corresponds to the nucleotide sequence of the polymorphism site contained in the polynucleotide.

According to this embodiment, there is provided a method of determining the polymorphism site contained in a predetermined polynucleotide, more typically, in a polymorphism gene, and simultaneously provided a method of quickly and simply determining the nucleotide sequence of the polymorphism site.

In the case where a target polymorphism gene is amplified by PCR, it is necessary to select a primer pair which sandwich a polymorphism site to be determined.

Subsequently, nucleic acid probes $PR_1$ to $PR_n$ (n is an integer of 2 or more) containing nucleotide sequences complementary to respective polymorphism sequences $PS_1$ to $PS_n$ are mixed with the test sample. Since nucleic acid probe $PR_1$ contains a nucleotide sequence complementary to polymorphism sequence $PS_1$, only nucleic acid probe $PR_1$ can be bound to a target polymorphism gene when the type of a target polymorphism gene is $PS_1$. Similarly, when target polymorphism genes are $PS_2$ to $PS_n$, they can bind to nucleic acid probes $PR_2$ to $PR_n$, respectively.

As long as an unknown polymorphism sequence is not present in the target polymorphism gene, any one of polymorphism sequences $PS_1$ to $PS_n$ is included in the polymorphism gene. Therefore, if the polymorphism gene is mixed with nucleic acid probes $PR_1$ to $PR_n$, any one of nucleic acid probes $PR_1$ to $PR_n$ comes to bind to a polymorphism site of the polymorphism gene.

Since each of the nucleotide probes $PR_1$ to $PR_n$ is labeled with a detectable marker substance, more preferably, with a fluorescent substance or luminescent substance, the polymorphism gene having the nucleic acid probe bound thereto can be detected by the detection operation described below.

The marker substances to be attached to nucleic acid probes may be the same in type. However, it is preferable that at least two types of marker substances be used so as to determine which nucleic acid probe binds to the target polymorphism gene. When a single type of marker substance is used alone, it is possible to determine which nucleic acid probe binds to the target polymorphism target gene by changing the length of each of nucleic acid probes.

After any one of nucleic acid probes $PR_1$ to $PR_n$ is bound to the target polymorphism gene, nucleic acid probes $PR_1$ to $PR_n$ present in a micro space are determined.

The target polymorphism gene present in the micro space can be detected by detecting the marker of the nucleic acid probe bound to the gene. As describe above, since the volume of the micro space is extremely small, the detection is preferably performed by using laser light. More specifically, the fluorescence in the micro space is detected under microscopic field of view.

The detection step may be performed by using a device, for example, shown in Example 2. Specifically in this embodiment, FCS may be used in order to find which nucleic acid probe is bound to the target allele in the manner mentioned below.

(1) laser light is applied to the micro space shown in FIG. 6B,
(2) the intensity of fluorescence emitted from a fluorescent substance present in the micro space is measured with time to obtain data shown in FIGS. 9A to 9D,
(3) calculating an expected value of a product I(t) $\times$ I(t+τ) which are fluorescent intensities of two different time points, to obtain an auto correlation function:

$$\text{Equation } G(\tau) = <I(t)I(T+\tau)>,$$

(4) The autocorrelation function obtained in the step (3) is analyzed by using the following equation 2:

$$G(\tau) = 1 + \frac{1}{N}\left[\left\{\frac{1-y}{1+\dfrac{\tau}{\tau_{unbound}}}\sqrt{\frac{1}{1+S^2 \cdot \dfrac{\tau}{\tau_{unbound}}}}\right\} + \left\{\frac{y}{1+\dfrac{\tau}{\tau_{bound}}}\sqrt{\frac{1}{1+S^2 \cdot \dfrac{\tau}{\tau_{bound}}}}\right\}\right] \quad \text{...(2)}$$

where N is an average number of fluorescent molecules;

$\tau_{unbound} \approx wo^2/4D_{unbound}$: translational diffusion time of a free nucleic acid probe;

$\tau_{bound} = wo^2/4D_{bound}$: translational diffusion time of a nucleic acid probe bound;

y is a ratio of the nucleic acid probe bound; and

s is wo/zo (wo is a diameter of a detection region; 2zo is a length of a region, $D_{unbound}$ and $D_{bound}$ are translational diffusion constants of a nucleic acid probe unbound and a nucleic acid probe bound), and

(5) autocorrelations before and after each of the nucleic acid probes is added.

To determine which nucleic acid probe is bound by FCS, for example, each of the nucleic acid probes may be distinguishably labeled with fluorescent markers different in excitation wavelength and/or fluorescent wavelength.

Furthermore, if the nucleic acid probes differs in size, the Brownian movement and the autocorrelation function differ. Therefore, it is possible to determine which nucleic acid probe is bound to a sequence by using the nucleic acid probes different in size. It is needless to say that the type of nucleic acid probe bound to a target allele may be determined by using the probes different in size and a fluorescent marker.

The target allele may be determined not only by a detection method using a hybridization reaction as an index but also by performing a PCR reaction using a labeled sequence which has been used as a probe, as a primer, and determining the type of marker of an amplified product. Furthermore, the PCR reaction may be used as an index. When such a PCR reaction is used as an index, if primers are designed so as to produce amplified products of different sizes depending upon types of polymorphism, the types of target allele can be determined based on the difference of autocorrelation functions ascribed to the different sizes of the amplified products.

As described in the foregoing, if the present invention is employed, a type of polymorphism site of a target allele can be very quickly and simply determined.

Determination of the type of HLA (see FIG. 10)

According to an aspect of the present invention shown in Example 3, there is provided a method of determining, for example, the type of HLA.

In this example, the type of polymorphism sequence showing an allo antigenicity to region DRB1 of HLA class II is determined. DRB1*15 and DRB1*16 are subtypes of DR2. DRB1*15 (Sequence Number 1) and DRB1*16 (Sequence Number 2) differ only in 141st nucleotide (T→C) and 180th nucleotide (G→C).

Probe 1, which has a nucleotide sequence complementary to the nucleotide sequence from the 141st to 180th nucleotide of DRB1*15, and probe 2, which has a nucleotide sequence complementary to the nucleotide sequence from the 141st to 180th nucleotide of DRB1*16, are prepared. Probe 1 is labeled with fluorescein isothiocyanate (FITC). Probe 2 is labeled with rhodamine. Each of probes 1 and 2 is added to a solution up to a concentration of about $10^{-8}$ M.

The nucleotide sequence of 60-200th nucleotides is amplified by PCR using a primer pair capable of specifically binding to a consensus region. Further, a solution is added up to a concentration of about $10^{-8}$ to prepare a DNA test sample.

A drop of the DNA test sample is placed on a surface of a cover glass. After probe 1 and probe 2 are added, a hybridization reaction is performed at a temperature of about 58 to 60°C.

A change in autocorrelation function of fluorescent luminescence is determined before and after the hybridization reaction.

For example, in the case where the type of test sample DNA is a DRB1*15 homotype, only probe 1 is bound. As a result, only the autocorrelation function of the yellow-green fluorescence emitted from FITC changes. On the other hand, in the case where the type of test sample DNA is a DRB1*16 homotype, only probe 2 is bound. As a result, only the autocorrelation function of the yellow-green fluorescence emitted from rhodamine changes. Furthermore, when the type of test sample DNA is a hetero of DRB1*15 and DRB1*16, probe 1 and probe 2 are bound. As a result, both autocorrelation functions of the fluorescence emitted from FITC and emitted from rhodamine change. If the type of test sample DNA is neither DRB1*15 nor DRB1*16, the autocorelation functions do not change at all.

Probe 1 and probe 2 are labeled with different marker substances as described above. Therefore, if they are added in the same vessel, it is possible to determine which probe binds to a test sample DNA. In view of the recent finding that numerous polymorphism portions are present in HLA, the method of the present invention has a great advantage because analysis can be made by adding many types of probes simultaneously to the same vessel.

In the method of this example, probes labeled with different marker substances are used. However, probes different in length may be used. In this case, each of probes must be selected so as to have the length which is large enough to determine the difference in fluctuation by FCS. For example, in the aforementioned examples in which DRB1*15 and DRB1*16 are separately recognized, probe 1 and probe 3 (Sequence number 3; longer than probe 1 by 20 nucleotides) may be used.

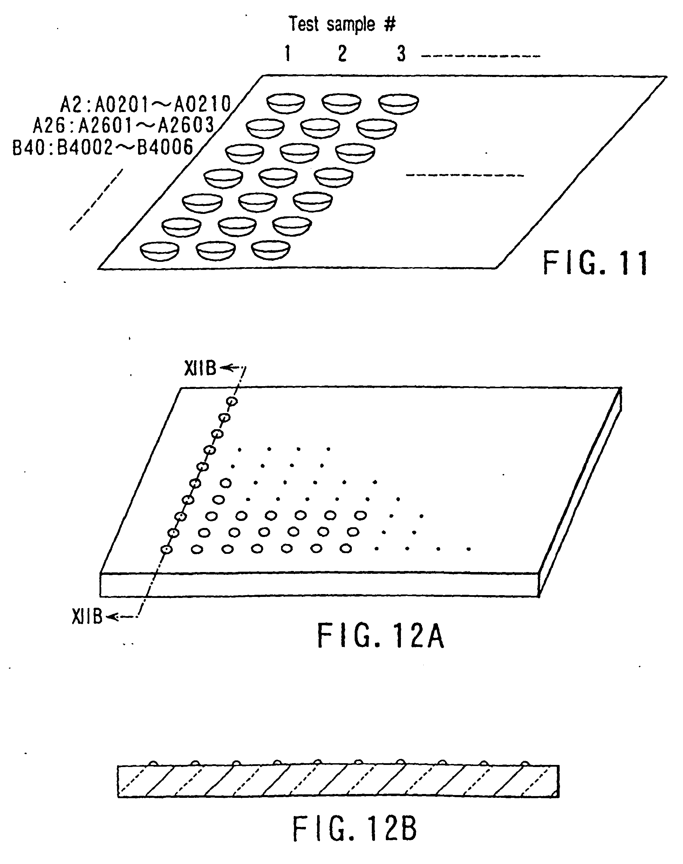

FIG. 11 shows an application example of the method of the present invention to a clinical test. According to the application example, the sub-class types of many polymorphism sequences can be quickly determined. Therefore, this method is usefully used to determine the type of an HLA since numerous polymorphism sites are present in the HLA and many subclasses are identified for each of the polymorphism sites.

As is apparent from FIG. 11, polymorphism sequences (A2, A26, B40 ·····) of an HLA are added to wells formed in a flat glass plate. More specifically, different polymorphism sequences are added to the wells of different columns. Thereafter, a probe group is added which is capable of binding specifically to subclasses of polymorphism sequences. Subsequently, which probe is bound is determined separately per well, as detailed in this example. In this manner, the subtypes of many polymorphism sequences can be determined. It is needless to say that that the polymorphism sequences to be added to the well are not limited to those of the HLA. Different polymorphism genes in type may be added to the wells.

According to the method of an example of the present invention, polymorphism sites of many types of polynucleotides can be quickly and easily determined.

Example 4

According to still another aspect of the present invention, it is possible to provide a method capable of analyzing a blood type, which is one of polymorphism.

This method can be performed, for example, as described below. First, an antigen/antibody reaction proceeds of a sample, is directly observed, if the movement of the labeled antibody is monitored by fluorescent correlation spectroscopy. If the state of labeled antibody is analyzed in this manner, it is possible to determine whether or not an antigen/antibody reaction takes place.

Thus, according to an aspect of the present invention, it is possible to determine blood types of blood cells, more specifically, a grouping test and a reverse grouping test with respect to red blood cell types, and to detect irregular antibodies, and anti-blood cell specific antigens and antibodies, and an antibodies of viruses and bacteria.

More specifically, to detect an antigen or an antibody as a detection target in a test sample, use is made of a fluorescent-substance labeled antibody or antigen which is specific to the antigen or antibody mentioned above. The antigen or antibody is first reacted with the fluorescent-substance labeled one. Subsequently, fluctuation of the fluorescence intensity of the fluorescent substance is measured with time. The obtained data is converted by a fluorescent autocorrelation function. As a result, the number and size of molecules labeled with a fluorescent substance can be determined.

In the method according to an embodiment of the present invention, it is not necessary to separate any substance present in a test sample, by an operation such as B/F separation from the beginning to the end of the determination process. In this example, the movement of the marker molecules is determined by continuously monitoring it with time starting from the time a sample and an arbitral reagent are added to a reaction vessel and the time period of a reaction taking place in the vessel. Therefore, it is possible to determine the movement of the marker molecules changed with time by the antigen/antibody reaction. Furthermore, the determination can be accurately performed under natural conditions which varies depending upon the antigen/antibody reaction proceeding in the reaction system mentioned above.

The method according to an embodiment of the present invention does not require steps other than a specific reaction, that is, a coagulation reaction step, washing step and a step of forming a centrifugal-reaction coagulation pattern which are performed in a conventional transfusion test. Furthermore, in this method, determination can be stated immediately after a sample and a reagent are mixed. Therefore, it is possible to reduce the examination time and simplify the examination step and lower occurrence of a nonspecific reaction, compared to a conventional method.

After the reaction step is performed as mentioned above, the detection step can be carried out by the device according to Example 2. The obtained data is analyzed as explained below. A fluorescent signal is measured for about one minute. The obtained fluorescent signals are sequentially stored in a memory portion and simultaneously applied to a fluorescent autocorrelation function G(t) to evaluate them. These procedures may be previously programmed.

The fluorescent autocorrelation function G(t) is performed in accordance with Equation 3 below based on an average number N of fluorescent molecules within a measurement region, translational time ($\tau_{mono}$) and translational time ($\tau_{poly}$) in accordance with a method of Rigler et al. (see Fluorescence Spectroscopy - new methods and applications, Springer Berlin, 13-24, In J. R. Ladowicz (Ed.), 1992). The time $\tau_{mono}$ is the translational time of free substrate molecule labeled with a marker, which serves as a nonreactive molecule including a fluorescent marker substance. The time $\tau_{poly}$ is the translational time of the labeled substrate molecule bound to a target molecule after an antigen/antibody reaction.

$$G(t) = 1 + \frac{1}{N}\left[\left\{\frac{1-y}{1+\dfrac{t}{\tau_{mono}}}\sqrt{\frac{1}{1+s^2\cdot\dfrac{t}{\tau_{mono}}}}\right\} + \left\{\frac{y}{1+\dfrac{t}{\tau_{poly}}}\sqrt{\frac{1}{1+s^2\cdot\dfrac{t}{\tau_{poly}}}}\right\}\right] \quad \dots (3)$$

In Equation 3, y is a ratio of a reaction component, $\tau_{mono}=Wo^2/4D_{mono}$, $\tau_{poly}=Wo^2/4D_{poly}$, $S=Wo/Zo$ (where, Wo is a diameter of a volume element of a nearly cylindrical measurement region formed in a micro field of vision near a focal point (see FIG. 3), and 2Zo refers to the length of the volume element). $D_{mono}$ and $D_{poly}$ are translational diffusion coefficients of a non-binding component and a binding component, respectively.

This example thus constituted finds applications in a wide variety of fields including not only methods related to a blood type described later but also determination tests for infectious diseases by viruses or bacteria and determination tests by means of an antigen-antibody reaction.

Furthermore, in this embodiment, a method of changing a molecular weight of an antibody by modifying a molecule with a protein which will not affect an antigen-antibody reaction. In this case, detection is made based upon the observation which antibody reagent (more specifically, which molecular-weight) has been changed in moving speed. Multiple items can be determined by using a single fluorescent substance.

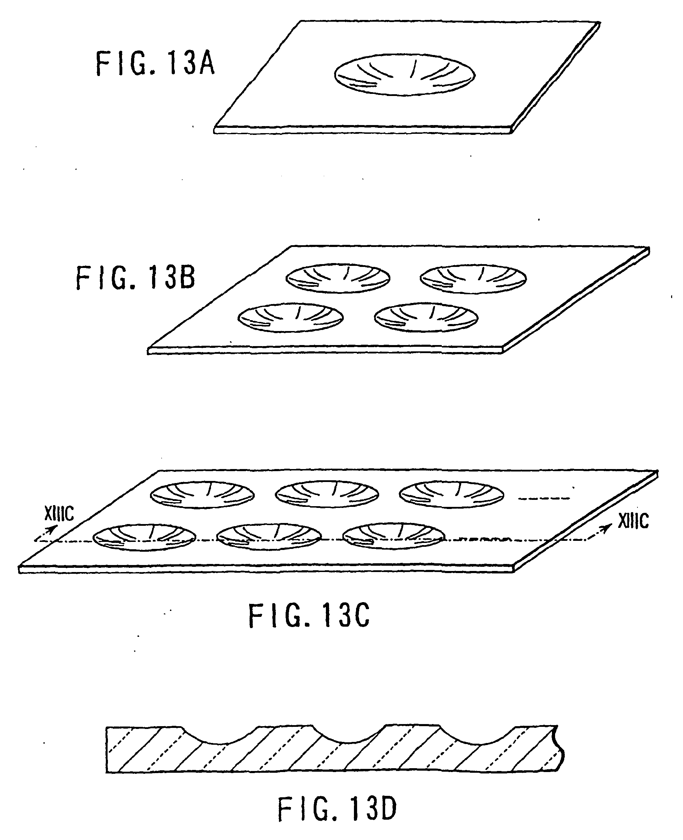

The reaction vessels which can be used in this example include the aforementioned slide glass on which a sample can be dotted (FIGS. 12A and 12B), a microplate having a plurality of micro wells (FIG. 11), and a plate having an appropriate number of micro-wells (FIGS. 13A and 13D). In this case, since only a small volume is required for a reaction, an ultra micro well plate having, for example, 384 wells is applicable. If the ultra micro well plate is used, a blood test can be performed at an extremely high speed and with a high throughput.

In the following example, FCS measurement was performed by using the FCS device shown in FIG. 5. To explain more specifically, a sample was attached in the form of a liquid drop onto a sample slide and placed on a commercially available device (ConfoCor, CarlZissJe naGmbH). The sample liquid drop was excited by a CW Ar+laser beam passing through an object lenz of $40\times$ magnification (C-Apochromat, NA=1.2). The resultant emission light was measured in the form of a fluorescent signal by an avalanche diode (APD), SPCM-200-PQ (manufactured by EG & G Co., Ltd.) in a single photon counting mode. The fluorescent signal thus measured was analyzed and evaluated by a digital corelater, ALV 5000/E (ALV GmbH). The volume of the sample near a focal point region was determined based on a diffusion coefficient of rhodamine 6G. Furthermore, the volume element was determined by using a concentration of fluorescein and a Flu-dUTP solution.

At this time, an average number of molecules and a translational diffusion coefficient in a detection area ($10^{-15}$L) were obtained. The embodiment below shows a method of identifying a blood type which includes the steps of detecting a time-dependence change of data before and after a specific reaction, converting the data, and detecting the size of molecules based on the diffusion speed shown in FIG. 9.

(1) Blood grouping test for determining a blood type of red blood cells

An antibody reagent labeled with fluorescence such as FITC was dispensed into a control well of a measurement vessel of a slide glass having a plurality of recesses, as shown in FIG. 13B. After that, measurement is immediately carried out. The volume of the measurement vessel may be set at, for example, about $10\mu l$, which is sufficient as a volume for FCS measurement region. A control well and a measurement well are not necessarily set separately. The measurements before and after addition of a reagent may be regarded as a control and a test group.

The concentration of red blood cells in a sample to be used may be low compared to that to be used in a general blood grouping test. For example, the red blood cell concentration is $10^3$ - $10^4$ cells/$\mu$L, more preferably, 2 to $5\times10^3$ cells/$\mu$L. Therefore, if this embodiment is applied to the blood-type test, the test can be carried out in a smaller volume of blood than a conventional test. To be more specific, the dilution factor of whole blood may be about 0.1%.

As a reaction system, other than the reaction system as shown in FIG. 13C, a system as shown in FIG. 13A may be used. In the system of FIG. 13C, an anti-A antibody and an anti-B antibody labeled with FITC are tested in separate wells, whereas an anti-A antibody labeled with FITC and an anti-B antibody labeled with Cy3 are mixed in the same well, in the system of FIG. 13A. Determination is made based upon a change in mobility of a fluorescent substance used as a marker. If the mobility changes, it is determined that the antibody has reacted with blood cells (positive reaction (+)). If the mobility does not change, it is determined that the antibody has not reacted with blood (negative reaction (-)).

Table 1

| | Blood type of blood cells tested | | | |
| --- | --- | --- | --- | --- |
| | A | B | O | AB |
| FITC (anti A) | + | - | - | + |
| Cy 3 (anti B) | - | + | - | + |

According to the reaction method, measurement can be made by only one step in which an antigen and an antibody are mixed. Therefore, incubation and centrifugal operation required for forming a coagulation pattern are not required. In addition, since the measurement region is extremely small, the amounts (volumes) of blood cells and an antibody reagent to be used in a reaction may be small. As a result, the reaction cost can be reduced. Furthermore, the reaction between a single antigen and a single antibody can be directly captured simply based upon a change in mobility of a fluorescent molecule without using a solid surface and a coagulation pattern. Therefore, there is less chance of causing a nonspecific reaction. Hence, quantitational determination can be made by using a numerical measurement value. This is an important feature in solving a problem of a transfusion test, that is, determination of a blood subtype.

Furthermore, as shown in FIG. 13A, a plurality of items with respect to a single test sample can be determined in the same vessel. It is therefore possible to reduce the measurement time and the amounts of test samples and reagents, etc.

Furthermore, the sample of the reaction system may be stirred by an appropriate means such as an ultrasonic wave.

(2) Reverse grouping test for determining a blood type of red blood cells

After the standard blood cells of A type and B type and a serum to be tested are mixed, the mixture is added to the well in which a standard antibody labeled with fluorescence has been dispensed. Immediately upon mixing, measurement is immediately performed. In this case, the determination is principally performed based upon a competitive reaction of an anti-blood type antibody present in a test serum and the standard antibody.

By using different fluorescent substances in the same as in the grouping test, the standard blood cells of both A type and B type and fluorescence-labeled standard antibodies of both anti A type and anti B type are mixed in the same well per test sample and subjected to a reaction.

Determination is made based upon a change in mobility of a fluorescent substance serving as a marker. If the mobility changes, it is determined that the antibody has reacted with blood cells (positive reaction (+)). If the mobility does not change, it is determined that the antibody has not reacted with the blood cells (negative reaction (-)).

Table 2

| | Blood type of blood cells tested | | | |
| --- | --- | --- | --- | --- |
| | A | B | O | AB |
| FITC (anti A) | - | + | - | + |
| Cy 3 (anti B) | + | - | - | + |

(3) Screening test for irregular antibody

To perform a screening test for irregular antibodies to red blood cells, a fluorescence-labeled anti human globulin serum serving as a secondary antibody is previously dispensed in a reaction well. Subsequently, the standard blood cells, that is, two or three types of O-type normal human blood cells (containing sufficient antigens for determining clinically important antibodies) are generally mixed with the serum to be tested. Immediately upon mixing, the resultant mixture is dispensed in reaction wells and subjected to detection.

The fluorescence-labeled serum can be bound to globulin other than irregular antibodies liberated in the test serum, changing the mobility of fluorescent substance. However, when the irregular antibody bound to the standard blood cells is present, the mobility of the fluorescent substance changes more significantly than the case mentioned above. Hence, the presence of the irregular antibody can be easily determined.

(4) Application

The embodiments mentioned above can be applied to the case where an antibody is identified after screening. In this case, the antibody is identified by using panel blood cells for antibody identification which are prepared by distributing clinically important antigens as panels. To explain more specifically, the antibody is identified by reacting the panel blood cells in a separate well and analyzing its reaction pattern.

Furthermore, when the reaction using the standard blood cells is employed, if desired, the standard blood cells may be immobilized onto a reaction vessel. Alternatively, it may be possible to use a reaction vessel having a partition which prevents blood cells from moving in the micro field of view. With this structure, the background may be minimized.

The entire contents of the document(s) cited herein are incorporated by reference in the beginning of this text.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

Industrial applicability

The present invention is useful for analyzing a disease-related gene for use in clinical treatments such as gene treatment and diagnosis, for analyzing a polymorphism in blood transfusion and organ transplantation. The present invention is also useful in studying basic polymorphism and disease-related genes.

**Claims**

1. A method of detecting a polymorphism site, comprising:

     (1) reacting, in a same vessel, a test sample containing a polymorphism site with a plurality of types of probes corresponding to a plurality of types of the polymorphism site to be identified of said test sample, said probes binding to said plurality of types of the polymorphism site with a high affinity and being labelled with marker substances so as to optically distinguish from each other; and
     (2) optically measuring and analyzing a positional change of the marker substances by micro-movement at a plurality of time points in the course of the reaction, thereby detecting the types of polymorphism sites of said test sample.

2. The method according to claim 1, wherein said test sample is a test sample DNA fragment containing a sequence of a polymorphism site, which test sample is reacted to hybridize with a plurality of types of DNA probes respectively having sequences complementary to a plurality of polymorphism sequences to be identified and contained in the test sample DNA fragment.

3. The method according to claim 2, wherein the marker substances of the plurality of types of DNA probes respectively are attached to the nucleotide corresponding to the polymorphism sites and wherein a nucleic acid synthetase having a repair function is reacted to a hybridized product obtained prior to measuring and analyzing a positional change of the marker substances.

4. The method according to claim 3, wherein said nucleic acid synthetase is an enzyme having an exonuclease activity.

5. The method according to any one of claims 1 to 4, wherein the polymorphism site is a single nucleotide polymorphism.

6. The method according to claim 1, wherein said test sample contains a polynucleotide containing a polymorphism site having a polymorphism sequence selected from $PS_1$ to $PS_n$ and said plurality of types of probes are DNA probes $PR_1$ to $PR_n$ containing nucleotide sequences complementary to respective polymorphism sequences $PS_1$ to $PS_n$, and wherein said positional change of the marker substance is measured in a microspace of from $10^{-21}$ to $10^{-3}$ L to determine which one of the DNA probes $PR_1$ to $PR_n$ binds to the polynucleotide.

**7.** The method according to claim 6, wherein said polynucleotide is a gene for a human histocompatible antigen.

**8.** The method according to claim 1, wherein said plurality of types of probes are a plurality of antibodies respectively having a specificity to a plurality of antigens contained in the test sample, said marker substances being fluorescent substances.

**9.** The method according to claim 8, wherein the polymorphism site is formed of a protein.

**10.** The method according to claim 8, wherein the test sample is red blood cells and said antigen is a surface-layer antigen of red blood cells.

**11.** A method of detecting a polymorphism site, comprising:

(1) placing, in a same vessel, a test sample, a plurality of types of antigens respectively having a specificity to a plurality of antibodies to be identified contained in the test sample, and a plurality of antibodies which compete with said antibodies to be identified for said antigens and which are labelled with marker substances so as to optically distinguish from each other; and
(2) optically measuring and analyzing a positional change of the marker substances by micro-movement at a plurality of time points in the course of the reaction, thereby detecting the polymorphism site.

**12.** The method according to claim 11, wherein the test sample is a body fluid derived from an organism.

**13.** The method according to any one of claims 1 to 6, 8 and 11, wherein said marker substances are fluorescent substances, said detecting is performed by a confocal microscope, and said analyzing is performed by a fluorescent correlation spectroscopy.

**14.** The method according to any one of claims 1 to 13, wherein said optical determining includes measuring fluctuation of the marker substances.

**Patentansprüche**

**1.** Verfahren zum Nachweis eines Polymorphismusortes, umfassend:

(1) Umsetzen, im gleichen Reaktionsgefäß, einer Testprobe, die einen Polymorphismusort enthält, mit einer Vielzahl von Sondentypen, die einer Vielzahl von zu identifizierenden Typen des Polymorphismusortes dieser Testprobe entsprechen, wobei diese Sonden mit einer hohen Affinität an die Vielzahl von Typen des Polymorphismusortes binden und so mit Markersubstanzen markiert sind, dass sie sich optisch voneinander unterscheiden; und
(2) optisches Messen und Analysieren einer Positionsänderung der Markersubstanzen durch Mikrobewegung zu einer Vielzahl von Zeitpunkten im Verlauf der Reaktion und dadurch Nachweisen der Typen von Polymorphismusorten der Testprobe.

**2.** Verfahren nach Anspruch 1, wobei die Testprobe ein Testproben-DNA-Fragment ist, das eine Sequenz eines Polymorphismusortes enthält, wobei die Testprobe zum Hybridisieren mit einer Vielzahl von DNA-Sondentypen umgesetzt wird, die jeweils Sequenzen besitzen, die zu einer Vielzahl von zu identifizierenden und in dem Testproben-DNA-Fragment enthaltenen Polymorphismussequenzen komplementär sind.

**3.** Verfahren nach Anspruch 2, wobei die Markersubstanzen der Vielzahl von DNA-Sondentypen jeweils an das Nukleotid gebunden sind, das den Polymorphismusorten entspricht, und wobei man vor Messen und Analysieren einer Positionsänderung der Markersubstanzen eine Nukleinsäuresynthetase mit einer Repair-Funktion mit einem erhaltenen hybridisierten Produkt reagieren lässt.

**4.** Verfahren nach Anspruch 3, wobei die Nukleinsäuresynthetase ein Enzym mit einer Exonukleaseaktivität ist.

**5.** Verfahren nach irgendeinem der Ansprüche 1 bis 4, wobei der Polymorphismusort ein Einzelnukleotidpolymorphismus ist.

**6.** Verfahren nach Anspruch 1, wobei die Testprobe ein Polynukleotid enthält, das einen Polymorphismusort mit einer Polymorphismussequenz enthält, die ausgewählt ist aus $PS_1$ bis $PS_n$, und die Vielzahl von Sondentypen DNA-Sonden $PR_1$ bis $PR_n$ sind, die Nukleotidsequenzen enthalten, die zu den jeweiligen Polymorphismussequenzen $PS_1$ bis $PS_n$ komplementär sind, und wobei die Positionsänderung der Markersubstanz in einem Mikroraum von $10^{-21}$ bis $10^{-3}$ L gemessen wird, um festzustellen, welche der DNA-Sonden $PR_1$ bis $PR_n$ an das Polynukleotid bindet.

**7.** Verfahren nach Anspruch 6, wobei das Polynukleotid ein Gen für ein humanes Histokompatibilitätsantigen ist.

**8.** Verfahren nach Anspruch 1, wobei die Vielzahl von Sondentypen eine Vielzahl von Antikörpern ist, die jeweils für ein Vielzahl von in der Testprobe enthaltenen Antigenen spezifisch sind, wobei die Markersubstanzen fluoreszierende Substanzen sind.

**9.** Verfahren nach Anspruch 8, wobei der Polymorphismusort von einem Protein gebildet wird.

**10.** Verfahren nach Anspruch 8, wobei die Testprobe rote Blutzellen sind und das Antigen ein Oberflächenantigen von roten Blutzellen ist.

**11.** Verfahren zum Nachweis eines Polymorphismusortes, umfassend:

(1) Einbringen einer Testprobe, einer Vielzahl von Antigentypen, die jeweils für eine Vielzahl von in der Testprobe enthaltenen zu identifizierende Antikörper spezifisch sind, und einer Vielzahl von Antikörpern, die mit den zu identifizierenden Antikörpern um die Antigene konkurrieren und die so mit Markersubstanzen markiert sind, dass sie sich optisch voneinander unterscheiden; und
(2) optisches Messen und Analysieren einer Positionsänderung der Markersubstanzen durch Mikrobewegung zu einer Vielzahl von Zeitpunkten im Verlauf der Reaktion und dadurch Nachweisen des Polymorphismusortes.

**12.** Verfahren nach Anspruch 11, wobei die Testprobe eine Körperflüssigkeit ist, die aus einem Organismus stammt.

**13.** Verfahren nach irgendeinem der Ansprüche 1 bis 6, 8 and 11, wobei die Markersubstanzen fluoreszierende Substanzen sind, der Nachweis mit einem konfokalen Mikroskop erfolgt und die Analyse mit Fluoreszenzkorrelationsspektroskopie erfolgt.

**14.** Verfahren nach irgendeinem der Ansprüche 1 bis 13, wobei die optische Bestimmung die Messung der Fluktuation der Markersubstanzen beinhaltet.

**Revendications**

**1.** Procédé pour détecter un site polymorphe, comprenant :

(1) la réaction, dans un même récipient, d'un échantillon d'essai contenant un site polymorphe avec une pluralité de types de sondes correspondant à une pluralité de types du site polymorphe à identifier dudit échantillon d'essai, lesdites sondes se liant à ladite pluralité de types du site polymorphe avec une affinité élevée et étant marquées avec des substances marqueur de façon à les distinguer les unes des autres optiquement ; et
(2) la mesure et l'analyse optiques d'un changement positionnel des substances marqueur par micromouvement à une pluralité d'instants au cours de la réaction, détectant ainsi les types de sites polymorphes dudit échantillon d'essai.

**2.** Procédé selon la revendication 1, dans lequel ledit échantillon d'essai est un fragment d'ADN échantillon d'essai contenant une séquence d'un site polymorphe, lequel échantillon d'essai est mis à réagir pour s'hybrider avec une pluralité de types de sondes d'ADN possédant respectivement des séquences complémentaires à une pluralité de séquences polymorphes à identifier et contenues dans le fragment d'ADN échantillon d'essai.

**3.** Procédé selon la revendication 2, dans lequel les substances marqueur de la pluralité de types de sondes d'ADN sont respectivement attachées au nucléotide correspondant aux sites polymorphes et dans lequel une acide nucléique synthétase ayant une fonction de réparation est mise à réagir avec un produit hybridé obtenu avant de mesurer et d'analyser un changement positionnel des substances marqueur.

**4.** Procédé selon la revendication 3, dans lequel ladite acide nucléique synthétase est une enzyme possédant une activité d'exonucléase.

**5.** Procédé selon l'une quelconque des revendications 1 à 4, dans lequel le site polymorphe est un polymorphisme de nucléotide unique.

**6.** Procédé selon la revendication 1, dans lequel ledit échantillon d'essai contient un polynucléotide contenant un site polymorphe possédant une séquence polymorphe choisie parmi $PS_1$ à $PS_n$ et ladite pluralité de types de sondes se compose des sondes d'ADN $PR_1$ à $PR_n$ contenant des séquences nucléotidiques complémentaires aux séquences polymorphes $PS_1$ à $PS_n$ respectives, et dans lequel ledit changement positionnel de la substance marqueur est mesuré dans un micro-espace allant de $10^{-21}$ à $10^{-3}$ L afin de déterminer laquelle des sondes d'ADN $PR_1$ à $PR_n$ se lie au polynucléotide.

**7.** Procédé selon la revendication 6, dans lequel ledit polynucléotide est un gène pour un antigène histocompatible humain.

**8.** Procédé selon la revendication 1, dans lequel ladite pluralité de types de sondes est une pluralité d'anticorps ayant respectivement une spécificité pour une pluralité d'antigènes contenus dans l'échantillon d'essai, lesdites substances marqueur étant des substances fluorescentes.

**9.** Procédé selon la revendication 8, dans lequel le site polymorphe est formé d'une protéine.

**10.** Procédé selon la revendication 8, dans lequel l'échantillon d'essai se compose de globules rouges et ledit antigène est un antigène de surface de globules rouges.

**11.** Procédé pour détecter un site polymorphe, comprenant :

(1) la mise en place, dans un même récipient, d'un échantillon d'essai, d'une pluralité de types d'antigènes ayant respectivement une spécificité pour une pluralité d'anticorps à identifier contenus dans l'échantillon d'essai, et une pluralité d'anticorps qui entrent en compétition avec lesdits anticorps à identifier pour lesdits antigènes et qui sont marqués avec des substances marqueur de façon à les distinguer les uns des autres optiquement ; et
(2) la mesure et l'analyse optiques d'un changement positionnel des substances marqueur par micromouvement à une pluralité d'instants au cours de la réaction, détectant ainsi le site polymorphe.

**12.** Procédé selon la revendication 11, dans lequel l'échantillon d'essai est un fluide corporel dérivé d'un organisme.

**13.** Procédé selon l'une quelconque des revendications 1 à 6, 8 et 11 dans lequel lesdites substances marqueur sont des substances fluorescentes, ladite détection est effectuée à l'aide d'un microscope confocal, et ladite analyse est effectuée à l'aide d'une spectroscopie à corrélation de fluorescence.

**14.** Procédé selon l'une quelconque des revendications 1 à 13, dans lequel ladite détermination optique comprend la mesure d'une fluctuation des substances marqueur.

FIG. 1

Single-nucleotide replaced site

FIG. 2

Probe I

Probe II

FIG. 3

Hybridization

Complete match

Enzymatic treatment

FIG. 4

Hybridization

Mismatch

Enzymatic treatment

FIG. 5

FIG. 6A

FIG. 6B

Laser beam

Laser beam

FIG. 7

FIG. 8

FIG. 9A

Fluorescent intensity I

Time (t)   FIG. 9B

FIG. 9C

Fluorescent intensity I

Time (t)   FIG. 9D

("-" represents a common nucleotide)

```
  BP#   60                    80              .              .
DRB1*15 CAGGATGAACGCGGTGGGTGCTGTTTAAGGAACCGGTAAAG //
DRB1*16 ---------------------------------------- //

                  140           .    160           .    180
DRB1*15 AGCAGAGAGTGTCTTTGGGGTGGAGGCTCCCAGGAGGAGGCGGCGCGGGCTGCGGTGCT //
DRB1*16 --------C---------------------------------------C----- //

Probe 1
Probe 2
Probe 3

        380           400           420
DRB1*15 AGGTTCCCAGTGCCCGCACCCCGCCCAGGGAGCCCCGGCCTGGTGGCGTCGCTGTCAGTGT
DRB1*16 ------------------------------------------------------------
```

FIG. 10

Test sample #

1    2    3  ------------

A2:A0201~A0210
A26:A2601~A2603
B40:B4002~B4006

FIG. 11

XIIB

XIIB

FIG. 12A

FIG. 12B

FIG. 13A

FIG. 13B

XIIIC

XIIIC

FIG. 13C

FIG. 13D